# EUROPEAN PATENT APPLICATION

(11) **EP 4 137 131 A1**
(43) Date of publication of application: **22.02.2023**
(21) Application number: 21306124.5
(22) Date of filing: 17.08.2021
(51) Int. Cl.: A61K 31/4045, A61P 7/06, C07D 209/00

(54) **NOVEL SEROTONIN ANALOGUES AND THEIR USES FOR TREATING IRON-ASSOCIATED DISORDERS**

(71) Applicant: Centre National de la Recherche Scientifique (CNRS), 75016 Paris (FR); Institut Gustave-Roussy, 94800 Villejuif (FR); Institut National de la Santé et de la Recherche Médicale (INSERM), 75013 Paris (FR); Institut Curie, 75005 Paris (FR); Université Paris Cité, 75006 Paris (FR)
(72) Inventor: RODRIGUEZ, Raphaël, 75004 PARIS (FR); COTE, Francine, 75012 PARIS (FR); COMAN, Tereza, 75015 PARIS (FR); DEBIEU, Sylvain, 75014 PARIS (FR); CANEQUE COBO, Tatiana, 75004 PARIS (FR); HERMINE, Olivier, 75014 PARIS (FR); MULLER, Sebastian, 94700 IVRY-SUR-SEINE (FR); FALABREGUE, Marion, 75014 PARIS (FR)
(74) Representative: Regimbeau

(57) **Abstract**

The present invention relates to novel serotonin analogues of formula (I): 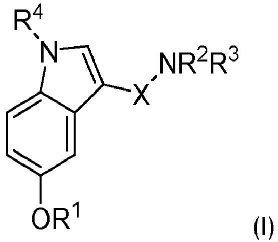 or a pharmaceutically acceptable salt and/or solvate thereof.

Another object of the present invention relates to the use of compound of formula (I) as a drug, in particular in the prevention and/or the treatment of iron overload- associated disorders.

## Description

### Field of the invention

The present invention relates to novel serotonin analogues and their use as drugs, in particular for preventing and/or treating iron-associated disorders.

### Background of the invention

Iron is essential for biological processes, in particular for the conduct of erythropoiesis, the production process of red blood cells.

Many diseases have iron associated disorders related to an ineffective erythropoiesis. In particular, the disorders may relate to an overload of iron in the body. Among the most common iron overload- associated disorders are iron-loading associated anemias such as β-thalassemia, myelodysplasia or hematopoietic stem-cell transplantation-related disorders.

β-thalassemia is a kind of genetic hemolytic anemia, related to an abnormal synthesis of β-hemoglobin, inducing apoptosis of erythroid progenitors. This disease affects 1,5% of the world's population, with a high prevalence in the poorest countries, in Africa and in India. It is responsible for 50 000 to 100 000 deaths per year. Current treatment consists in regular transfusions to maintain a normal hemoglobin level. However, repeated blood transfusions and hemoglobin apoptosis lead to an overload of iron in the organism causing high toxicity. It is thus essential to eliminate this excess of iron. Iron chelators, such as deferoxamine, may be used at this end and have been proved effective to reduce mortality. However, deferoxamine requires heavy treatment conditions such as daily injections and infusions. Oral iron chelators also exist such as deferiprone or deferasinox, for a more convenient use, but they are less effective. A new drug, the Luspatercept has been approved by FDA in 2019 for transfusion-dependent thalassemia. Luspatercept is a recombinant protein which has been shown to be effective in reducing anemia, decreasing transfusion requirements and lowering ferritin levels. However, the price of Luspatercept is particularly high, making it difficult to reach disadvantaged populations.

Myelodysplasia, or myelodysplastic syndrome (MDS) is a clonal hematopoietic disease characterized by anemia related to an inefficient hematopoiesis and progression to acute myeloid leukemia. This syndrome mainly affects people aged 60 and over. As for β-thalassemia, blood transfusions help to maintain a normal hemoglobin level but lead to an overload of iron in the organism. This overload of iron is also caused by the suppression of the production of hepcidin, a hormone regulating the iron metabolism in the body, due to this syndrome. Iron chelators are not recommended in view of their toxicity and the fragility of the patients. The Luspatercept has also been recently approved for the treatment of this disease.

Approximately 40,000 allografts are performed each year in the world, including about 2,500 in France, with an estimated growth rate of 7% per year. An allograft refers to a transplant wherein the donor and the recipient are two separate individuals. Hematopoietic stem cell allograft is an evolving technique that offers the prospect of a cure for hematologic malignancies (leukemias, lymphomas, myelomas) and other hematologic disorders (e.g., primary immune deficiency, bone marrow aplasia, myelodysplasia). Post-transfusion iron overload is relatively common in the context of hematopoietic stem cell transplantation. The use of iron chelators is very limited post allograft due to their toxicities. There is currently no alternative treatment to decrease post-transplant iron overload and help hematopoiesis so as to increase post-transplant survival.

There is therefore a need for alternative treatment of the iron-associated disorders, in particular for preventing and/or treating the overload of iron observed for example in β-thalassemia, MSD or in post-transplant patients, obtainable at a reasonable cost and with a satisfactory safety and efficacy profile, at least similar to the one of Luspatercept used in β-thalassemia and MSD.

Serotonin, also called 5-hydroxytryptamine (5-HT), is a neurotransmitter responding to the following formula:

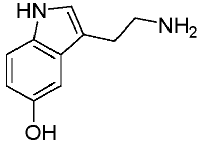

This molecule is essential for the metabolism, enabling to modulate mood, cognition, reward, learning, memory and numerous physiological processes such as vomiting and vasoconstriction. Serotonin is synthesized in neurons starting from tryptophan, an essential amino-acid brought to the brain through blood circulation. The rate limiting enzyme Tryptophan hydroxylase, responsible for serotonin synthesis is highly expressed in erythroid precursors and it has been shown that serotonin is synthesized at a critical transition checkpoint during erythroid progenitor's proliferation (Coman et al., Cell Reports, 2019, 26, 3246-3256). It has recently been demonstrated that the level of serotonin in the bone marrow directly impacts erythropoiesis. A high level of serotonin is able to enhance renewal of erythroid progenitors and thus to promote the production of red blood cells (Coman et la., Cell Reports, 2019). On the contrary, reduced levels of serotonin have been observed in patients suffering from myelodysplastic syndrome.

The present inventors assume that serotonin is able to modulate erythropoiesis by influencing the iron availability needed for the production of red blood cells. Serotonin thus represents an interesting therapeutic target for the treatment of anemia. However, serotonin has vasoconstriction/vasodilation properties via serotonin receptors, and thus cannot be injected.

To remedy the drawbacks of the existing treatments and based on the above hypothesis, the inventors have developed small serotonin analogues, easy to prepare, and able to act as iron chelators to normalize iron stores and make iron available for vital biological processes, which do not exhibit the toxicity observed in current iron chelators.

### Summary of the invention

In a first aspect, the present invention relates to compound of formula (I): 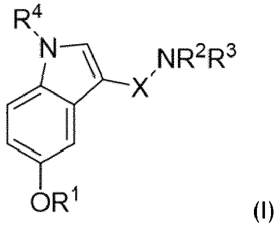 or a pharmaceutically acceptable salt and/or solvate thereof, wherein
R¹, R², R³ and R⁴ are independently selected in the group consisting of H, optionally substituted C₁-C₂₄alkyl, optionally substituted C₂-C₂₄ alkenyl, optionally substituted C₂-C₂₄ alkynyl, optionally substituted C₃-C₇ cycloalkyl, optionally substituted heterocycloalkyl, optionally substituted aryl and optionally substituted heteroaryl, provided that at least one of R¹, R² and R³ is not H, and
X is selected in the group consisting of C₁-C₁₂ alkyl, O-C₁-C₁₂ alkyl, C(O), C(O)-C₁-C₁₂ alkyl and NH-C(O)-C₁-C₁₂ alkyl.

In a second aspect, the present invention relates to a compound of formula (I) for use as a drug.

According to a third aspect, the present invention relates to a pharmaceutical composition comprising a compound of formula (I) and at least one pharmaceutically acceptable excipient.

A fourth aspect of the present invention lies in a pharmaceutical composition comprising a compound of formula (I) and at least one pharmaceutically acceptable excipient for use as a drug.

### Detailed Description

For the purpose of the invention, the term "pharmaceutically acceptable" is intended to mean what is useful to the preparation of a pharmaceutical composition, and what is generally safe and non-toxic, for a pharmaceutical use.

The term "pharmaceutically acceptable salt and/or solvate" is intended to mean, in the framework of the present invention, a salt and/or solvate of a compound which is pharmaceutically acceptable, as defined above, and which possesses the pharmacological activity of the corresponding compound.

The pharmaceutically acceptable salts comprise:
(1) acid addition salts formed with inorganic acids such as hydrochloric, hydrobromic, sulfuric, nitric and phosphoric acid and the like; or formed with organic acids such as acetic, benzenesulfonic, fumaric, glucoheptonic, gluconic, glutamic, glycolic, hydroxynaphtoic, 2-hydroxyethanesulfonic, lactic, maleic, malic, mandelic, methanesulfonic, muconic, 2-naphtalenesulfonic, propionic, succinic, dibenzoyl-L25 tartaric, tartaric, p-toluenesulfonic, trimethylacetic, and trifluoroacetic acid and the like, and
(2) base addition salts formed when an acid proton present in the compound is either replaced by a metal ion, such as an alkali metal ion, an alkaline-earth metal ion, or an aluminium ion; or coordinated with an organic or inorganic base. Acceptable organic bases comprise diethanolamine, ethanolamine, N-methylglucamine, triethanolamine, tromethamine and the like. Acceptable inorganic bases comprise aluminium hydroxide, calcium hydroxide, potassium hydroxide, sodium carbonate and sodium hydroxide.

Acceptable solvates of the compounds of the present invention include conventional solvates such as those formed during the last step of the preparation of the compounds of the invention due to the presence of solvents. As an example, mention may be made of solvates due to the presence of water (these solvates are also called hydrates) or ethanol.

The term "halogen", as used in the present invention, refers to a fluorine, bromine, chlorine or iodine atom.

The term "Cₓ-C_{y} alkyl", as used in the present invention, refers to a straight or branched monovalent saturated hydrocarbon chain containing from x to y carbon atoms including, but not limited to, methyl, ethyl, n-propyl, iso-propyl, n-butyl, iso-butyl, sec-butyl, t-butyl, n-pentyl, n-hexyl, and the like.

The term "Cₓ-C_{y} alkenyl", as used in the present invention, refers to a straight or branched monovalent unsaturated hydrocarbon chain containing from x to y carbon atoms and comprising at least one double bond including, but not limited to, ethenyl, propenyl, butenyl, pentenyl, hexenyl and the like.

The term "Cₓ-C_{y} alkynyl", as used in the present invention, refers to a straight or branched monovalent unsaturated hydrocarbon chain containing from x to y carbon atoms and comprising at least one triple bond including, but not limited to, ethynyl, propynyl (or propargyl), butynyl, pentynyl, hexynyl and the like.

The term "Cₓ-C_{y} haloalkyl" refers to a Cₓ-C_{y} alkyl chain as defined above wherein one or more hydrogen atoms are replaced by a halogen atom selected from fluorine, chlorine, bromine or iodine, preferably a fluorine atom. For example, it is a CF₃ group.

The term "cycloalkyl" refers to a saturated, non-aromatic, hydrocarbon ring, typically comprising from 3 to 7 carbons and comprising one or more fused or bridged ring(s) including, but not limited to, cyclopropyl, cyclobutyl, cyclopentyl, cyclohexyl, cycloheptyl, and the like.

The term "heterocycloalkyl" as used in the present invention refers to a non-aromatic, saturated or unsaturated monocycle or polycycle (comprising fused, bridged or spiro rings) comprising preferably 5 to 10, notably 5 or 6, atoms in the ring(s), in which the atoms of the ring(s) consist of carbon atoms and one or more, advantageously 1 to 4, and more advantageously 1 or 2, heteroatoms, such as a nitrogen, oxygen or sulphur atom, the remainder being carbon atoms. In particular, it can be an unsaturated ring, such as an unsaturated 5 or 6-membered monocycle. Preferably it comprises 1 or 2 nitrogen(s), in particular one. A heterocycle can be notably piperidinyl, piperizinyl, pyrrolidinyl, pyrazolidinyl, imidazolidinyl, azepanyl, thiazolidinyl, isothiazolidinyl, oxazocanyl, thiazepanyl, benzimidazolonyl.

The term "aryl" refers to an aromatic hydrocarbon group preferably comprising from 6 to 12 carbon atoms and comprising one or more fused rings, such as, for example, a phenyl, a naphthyl or an anthracenyl group. Advantageously, it is a phenyl group.

The term "heteroaryl", as used in the present invention, refers to an aromatic group comprising one or several, notably one or two, fused hydrocarbon cycles in which one or several, notably one to four, advantageously one or two, carbon atoms each have been replaced with heteroatoms selected from a sulfur atom, an oxygen atom and a nitrogen atom, preferably selected from an oxygen atom and a nitrogen atom. Preferably, the heteroaryl contains 5 to 12 carbon atoms, notably 5 to 10. It can be a furyl, thienyl, pyrrolyl, pyridyl, benzofuranyl, benzopyrrolyl, benzothipohenyl, isobenzofuranyl, isobenzopyrrolyl, isobenzothiophenyl, oxazolyl, isoxazolyl, thiazolyle, isothiazolyl, imidazolyl, pyrazolyl, oxadiazolyl, thiadiazolyl, triazolyl, tetrazolyl, pyridazinyl, pyrimidinyl, pyrazinyl, triazinyl, quinolyl, isoquinolyl, quinoxalyl or indyl.

In the context of the present invention, "unsaturated" means that the hydrocarbon chain may contain one or more unsaturation(s), i.e. a double bond C=C, advantageously one.

In the context of the present invention, "optionally substituted" means that the group in question is optionally substituted with one or more substituents which may be selected in particular from halogen, C₁-C₆ alkyl, C₁-C₆ haloalkyl, aryl, oxo, NR^{a}R^{b}, COR^{c}, CO₂R^{d}, CONR^{e}R^{f}, OR^{g}, CN and NO₂ wherein R^{a} to R^{g} are, independently of one another, H, C₁-C₆ alkyl, C₁-C₆ haloalkyl or aryl, preferably H or C₁-C₆ alkyl.

The term "oxo" refers to the substituent of formula "C(=O)".

The term "pharmaceutical composition" is meant in the framework of the present invention a composition having preventive and curative properties towards cancers.

### Compound of formula (I)

Compounds of the present invention respond to the following formula (I): 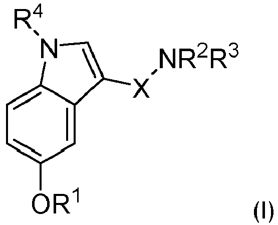 or a pharmaceutically acceptable salt and/or solvate thereof,
wherein
R¹, R², R³ and R⁴ are independently selected in the group consisting of H, optionally substituted C₁-C₂₄alkyl, optionally substituted C₂-C₂₄ alkenyl, optionally substituted C₂-C₂₄ alkynyl, optionally substituted C₃-C₇ cycloalkyl, optionally substituted heterocycloalkyl, optionally substituted aryl and optionally substituted heteroaryl, provided that at least one of R¹, R² and R³ is not H, and
X is selected in the group consisting of C₁-C₁₂ alkyl, O-C₁-C₁₂ alkyl, C(O), C(O)-C₁-C₁₂ alkyl and NH-C(O)-C₁-C₁₂ alkyl.

According to a particular embodiment, compound of formula (I) comprises at least one lipophilic group. The term « lipophilic group » (or » hydrophobic group ») refers to a chemical group which confers lipophilic properties to the compound of formula (I). Such lipophilic properties enhance the bioavailability of the compound by favorizing the passage though biological boundaries such as cell membranes, plasma membranes or lysosome. In particular, the lipophilic group is represented by a hydrocarbon group such as an aliphatic chain, linear or branched, saturated or unsaturated, comprising at least 3 carbon atoms, a cycloalkyl or an aromatic ring. Preferably, the lipophilic group according to the present invention corresponds to a C₂-C₁₂ alkyne, in particular to C₂-C₆ alkyne and notably to a propynyl group.

According to preferred embodiments, X is selected in the group consisting of C₁-C₆ alkyl, O-C₁-C₆ alkyl, C(O), C(O)-C₁-C₆ alkyl and NH-C(O)-C₁-C₆ alkyl. In particular, X is a C₁-C₆ alkyl. Preferably X is a methyl, an ethyl or a n-propyl, more preferably an ethyl.

According to preferred embodiments, R⁴ is selected in the group consisting of H, C₁-C₂₄ alkyl, C₂-C₂₄ alkenyl, C₂-C₂₄ alkynyl, C₃-C₇ cycloalkyl and aryl, said alkyl, alkenyl, alkynyl, aryl or cycloalkyl being optionally substituted with one or more halogens, C₁-C₆ alkyl, aryl, oxo, NH₂, CO₂H or OH. In particular, R⁴ is selected in the group consisting of H, C₁-C₁₂ alkyl and aryl. Preferably, R⁴ is H.

In preferred embodiments, R¹, R² and R³ are independently selected in the group consisting of H, C₁-C₂₄ alkyl, C₂-C₂₄ alkenyl, C₂-C₂₄ alkynyl, C₃-C₇ cycloalkyl and aryl, said alkyl, alkenyl, alkynyl, aryl or cycloalkyl being optionally substituted with one or more halogens, C₁-C₆ alkyl, aryl, oxo, NH₂, CO₂H or OH, provided that at least one of R¹, R² and R³ is not H. In particular, R¹, R² and R³ are independently selected in the group consisting of H, C₁-C₁₂ alkyl, C₂-C₁₂ alkenyl and C₂-C₁₂ alkynyl, said alkyl, alkenyl or alkynyl being optionally substituted with one or more halogens, C₁-C₆ alkyl, aryl, oxo, NH₂, CO₂H or OH, provided that at least one of R¹, R² and R³ is not H. Preferably, R¹, R² and R³ are independently selected in the group consisting of H, C₁-C₆ alkyl, C₂-C₆ alkenyl and C₂-C₆ alkynyl. In particular, R¹, R² and R³ are independently selected in the group consisting of H and C₂-C₆ alkynyl.

According to more preferred embodiments, R¹, R² and R³ are as defined above, provided that at least one of R¹, R² and R³ is an optionally substituted C₂-C₁₂ alkynyl, preferably a C₂-C₁₂ alkynyl optionally substituted with one or more halogens, C₁-C₆ alkyl, aryl, oxo, NH₂, CO₂H or OH. More preferably, at least one of R¹, R² and R³ is C₂-C₆ alkynyl.

Preferably, when R¹, R² and R³ is an alkynyl, it is preferably an ethynyl, a propynyl or a butynyl, notably a propynyl group.

According to a preferred embodiment, the present invention relates to the following compounds of formula (I):

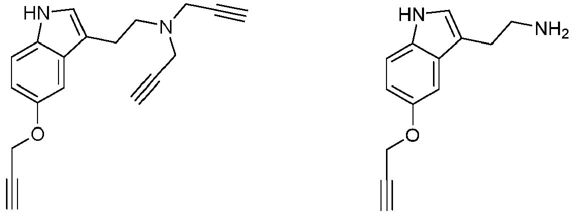

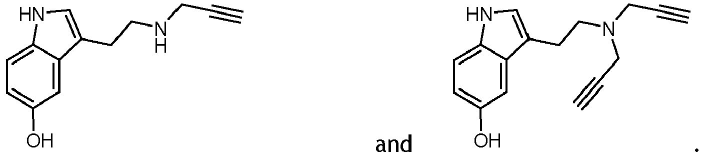

### Pharmaceutical composition

The present invention also relates to a pharmaceutical composition comprising at least one compound of formula (I) or a pharmaceutically acceptable salt and/or solvate thereof, and at least one pharmaceutically acceptable excipient.

Contrary to serotonin, compounds of the present invention do not link serotonin receptors responsible for vasoconstriction and vasodilation properties, so that compounds of formula (I) are injectable.

The pharmaceutical compositions of the invention can thus be intended to oral or parenteral (e.g., subcutaneous, intramuscular, intravenous) administration, preferably intravenous administration. The active ingredient can be administered in unit forms for administration, mixed with conventional pharmaceutical carriers, to animals, preferably mammals including humans.

For oral administration, the pharmaceutical composition can be in a solid or liquid (solution or suspension) form.

A solid composition can be in the form of tablets, gelatin capsules, powders, granules and the like. In tablets, the active ingredient can be mixed with pharmaceutical vehicle(s) such as gelatin, starch, lactose, magnesium stearate, talc, gum arabic and the like before being compressed. The tablets may be further coated, notably with sucrose or with other suitable materials, or they may be treated in such a way that they have a prolonged or delayed activity. In powders or granules, the active ingredient can be mixed or granulated with dispersing agents, wetting agents or suspending agents and with flavor correctors or sweeteners. In gelatin capsules, the active ingredient can be introduced into soft or hard gelatin capsules in the form of a powder or granules such as mentioned previously or in the form of a liquid composition such as mentioned below.

A liquid composition can contain the active ingredient together with a sweetener, a taste enhancer or a suitable coloring agent in a solvent such as water. The liquid composition can also be obtained by suspending or dissolving a powder or granules, as mentioned above, in a liquid such as water, juice, milk, etc. It can be for example a syrup or an elixir.

For parenteral administration, the composition can be in the form of an aqueous suspension or solution which may contain suspending agents and/or wetting agents. The composition is advantageously sterile. It can be in the form of an isotonic solution (in particular in comparison to blood).

The compounds of the invention can be used in a pharmaceutical composition at a dose ranging from 0.01 mg to 1,000 mg a day, administered in only one dose once a day or in several doses along the day, for example twice a day in equal doses. The daily administered dose is advantageously comprised between 5 mg and 500 mg, and more advantageously between 10 mg and 200 mg. However, it can be necessary to use doses out of these ranges, which could be noticed by the person skilled in the art.

The pharmaceutical compositions of the present invention may further comprise an additional therapeutic agent, notably useful in the treatment of iron-associated disorders, such as anemias. Preferably, this therapeutic agent is selected in the group consisting of Erythropoiesis-Stimulating Agents (ESAs) to activate the erythropoietin receptor and stimulate the bone marrow to make more red blood cells, such as Recombinant erythropoietin drugs as for example Luspatercept.

### Treatment

The compound of formula (I) or a pharmaceutically acceptable salt and/or solvate thereof, or the pharmaceutical composition according to the present invention are useful as a drug, notably in the prevention and/or treatment of iron-associated disorders.

The present invention thus relates to compound of formula (I) for use as a drug, in particular for use in the prevention and/or in the treatment of iron-associated disorders. The present invention also relates to a pharmaceutical composition according to the present invention for use as a drug, in particular for use in the prevention and/or in the treatment of iron-associated disorders.

The present invention also relates to the use of a compound of formula (I) according to the invention or a pharmaceutically acceptable salt and/or solvate thereof, or a pharmaceutical composition according to the present invention for the prevention and/or the treatment of iron-associated disorders.

The present invention also relates to a method for preventing and/or treating iron-associated disorders comprising the administration to a patient in need thereof of an effective dose of a compound of formula (I) according to the invention or a pharmaceutically acceptable salt and/or solvate thereof, or a pharmaceutical composition according to the present invention.

According to preferred embodiments, the iron overload-associated disorders are iron overload-associated disorders, notably selected among HFE-related hematochromatosis, non HFE-related hematochromatosis, congenital atransferrinenemia, iron-loading associated anemias, chronic liver diseases, chronic inflammation linked to cancer, autoimmune or inflammatory diseases, neurodegeneration with brain iron accumulation- associated diseases and polygenic neurodegenrative- associated diseases.

HFE-related hematochromatosis may notably be due to C282Y homozygosity or C282/H63D heterozygosity. Non HFE-related hematochromatosis include for example juvenile hemochromatosis type 2A or 2B, or may be due to Mutated transferrin receptor 2 or Mutated ferroportin 1 gene.

Neurodegeneration with brain iron accumulation- associated diseases include aceruloplasminemia, neuroferritinopathy, pantothenate kinase-associated neurodegenration, Wilson's disease and Beta-propeller Protein-Associated Neurodegeneration (BPAN).

Polygenic neurodegenerative disorders include Parkinson's disease and Alzheimer's disease. In particular, the iron overload-associated disorder is iron-loading related anemia, such as thalassemia, myelodysplasy, aplastic anemia, Blackfan diamond anemia, congenital dyserythopoietic anemia, chronic hemoytic anemia, in particular sickel cell disease, hematopoietic stem-cell transplantation-related disorder and chronic liver disease including viral hepatitis, alcoholic hepatitis, steatohepatitis (NASH), dysmetabolic iron overload syndrome.

According to a specific embodiment, the iron-loading associated anemia is thalassemia, myelodysplasia or hematopoietic stem-cell transplantation-related disorder.

### Method of preparation of compound of formula (I)

The compounds of the present invention may be prepared according to any method known by the skilled person in the art. In particular, they may be prepared by the following method.

A method for preparing compound of formula (I) according to the present invention comprises the steps of:
(i) reacting serotonin hydrochloride with a base, then
(ii) reacting the resulting serotonin with a R¹ group precursor, a R² group precursor and/or a R³ group precursor, when respectively R¹, R² and/or R³ is/are different from H.

In step (i), serotonin hydrochloride is typically dissolved in a solvent, notably an apolar aprotic solvent, including, but not limited to tetrahydrofuran (THF), diethylether (Et₂O), dimethylether (DME), dichloromethane, hexane, 1-4-dioxane, toluene and chloroform, or a polar aprotic solvent such as acetonitrile, pyridine, acetone, DMSO or acetic anhydride.

According to particular embodiments, the base is Na₂CO₃, K₂CO₃, NaOH, KOH, Ca(OH)₂, Ba(OH)₂, NaH, KH or LiOH, in particular NaH.

A Rⁿ group precursor (n being 1, 2 or 3) is understood, in the context of the present invention, as a compound able to react with deprotonated serotonin to insert a Rⁿ group on the serotonin so as to obtain a compound of formula (I). Typically, this precursor comprises a Rⁿ group attached to a leaving group such as a halide, in particular bromide or chloride, sulfonate esters, such as mesylate, tosylate or triflate.

In step (ii), when R¹, R² or R³ is H, serotonin does not react with the corresponding precursor of R¹ group, R² group or R³ group.

The reaction is typically conducted under inert atmosphere such as nitrogen (N₂) or argon (Ar) atmosphere.

Optionally, additional steps of protection/deprotection and/or of functionalization well-known from the skilled person in the art may occur before step (i) to protect the position that should not react. For example, the primary amine of serotonin may be protected to enable selective transformation of the OH group into OR¹ group.

The compound obtained can be separated from the reaction medium by methods well known to the person skilled in the art, such as by extraction, evaporation of the solvent or by precipitation or crystallisation (followed by filtration).

The compound can be also purified, if necessary, by methods well known to the person skilled in the art, such as by recrystallisation, by distillation, by chromatography on a column of silica gel or by high performance liquid chromatography (HPLC).

### Description of the figures

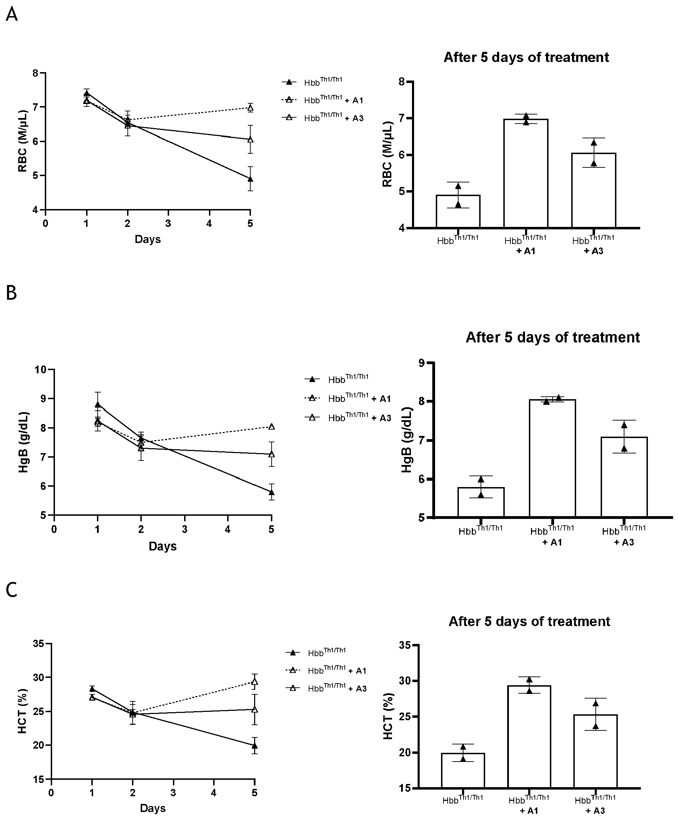

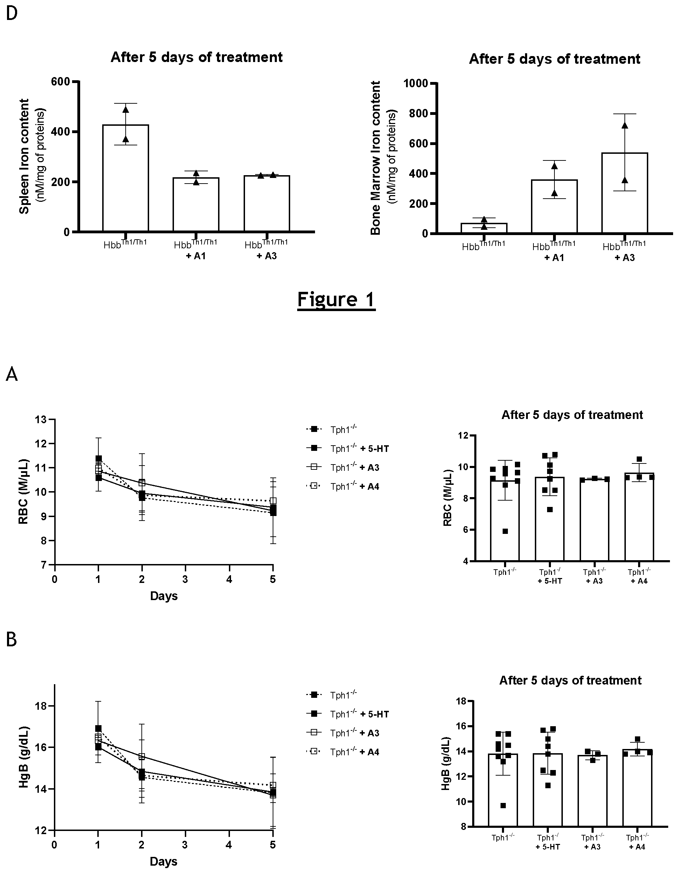

**Figure 1****: red blood cells production and iron overload on Model 1 (Hbb^{Th1/th1} mice**). Evolution from 1 to 5 days of the red blood count (A), hemoglobin rate (B), hematocrit contents (C), iron content in spleen (D, left) and in bone marrow (D, right) in Hbb^{Th1/th1} mice that received no dose or either analogue A1 or analogue A3.

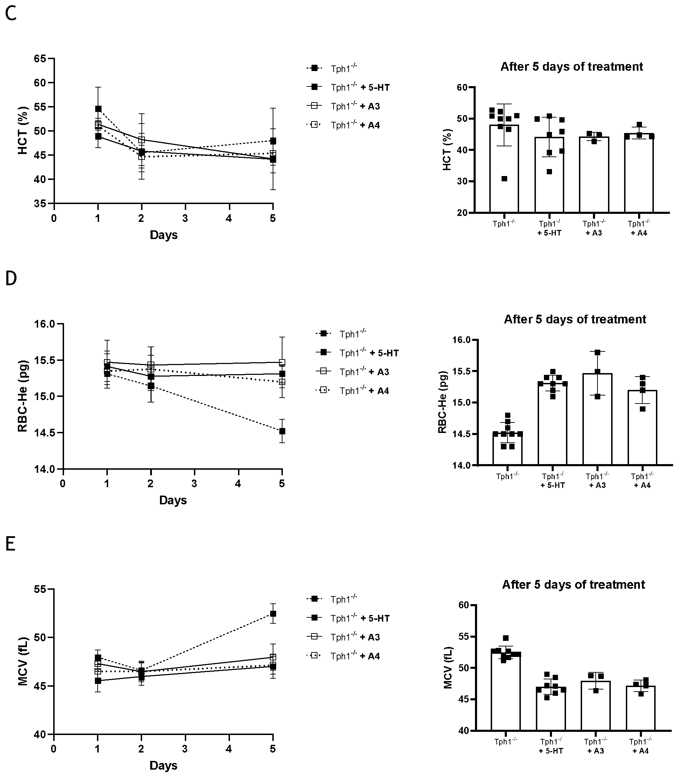

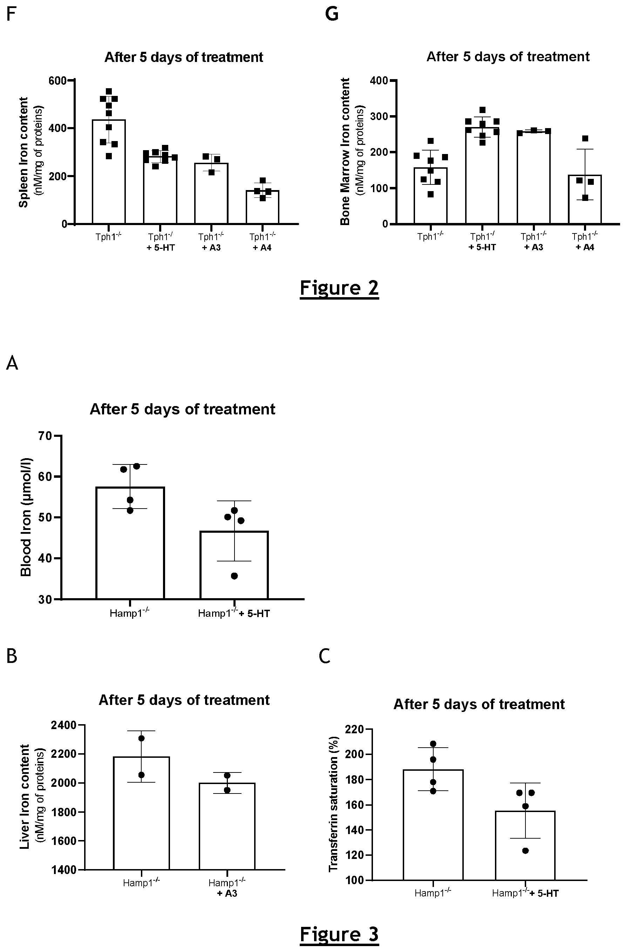

**Figure 2****: red blood cells production and iron overload on Model 2 (Tph1 KO mice).** Evolution from 1 to 5 days of the red blood count (A), hemoglobin rate (B), hematocrit contents (C), heme content in red blood cells (D), mean cellular volume (MCV) (E), iron content in spleen (F) and in bone marrow (G) in Tph1 KO mice that received no dose or either serotonin (5-HT), analogue A3 or analogue A4.
**Figure 3****: iron overload on Model 3 (Hamp KO).** Measure of iron content in blood (A) and transferrin saturation in % (C) after 5 days in Hamp KO mice that received no dose or serotonin. Measure of iron content in liver (B) after 5 days in Hamp KO mice that received no dose or A3 analogue.

### Examples

### 1. Synthesis

### Material and methods

### UPLC system:

Column: Aquity UPLC^{®} BEH C18 1.7 µm, 2.1x50 mm.
System: ACN (+0.1% FA) and MilliQ Water (+0.1% FA): isocratic at 5% of ACN (0.2 min), then linear gradient from 5% to 100% of ACN in 2.3 min, then isocratic at 100% of ACN (0.5 min).

### Synthesis and characterization

### Procedure for the synthesis of A1, A3 and A4:

Under inert atmosphere, serotonin hydrochloride (100 mg, 0.470 mmol, 1 eq.) was dissolved in THF (5 mL). NaH (36 mg, 0.893 mmol, 1.9 eq.) was added to the mixture. The mixture is stirred for 30 min, then propargyl bromide (57 µL, 0.517 mmol, 1.1 eq.) was added. The mixture was stirred for 3.5h, then was quenched with water. The resulting solution was extracted with DCM, dried on MgSO₄ and concentrated. The crude was purified by flash chromatography using DCM / MeOH (99/1 to 80/20) as eluent. 4 fractions were obtained and purified by preparative HPLC to give compounds A1, A3 and A4 after lyophilization.

### Characterization:

### Analogue A1 (N-(prop-2-yn-1-yl)-N-(2-(5-(prop-2-yn-1-yloxy)-1H-indol-3-yl)ethyl)prop-2-yn-1-amine):

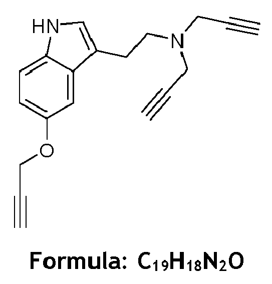

### UPLC: R_{T}: 1.88

¹H NMR (DMSO-*d6*, 500 MHz): 10.65 (1H, s); 7.24 (1H, d, J *9*.*1 Hz);* 7.11 (2H, d, J *18.3 Hz);* 6.76 (1H, dd, *J 8.75* & *1.75 Hz);* 4.75 (2H, d, J *1.7 Hz);* 3.50 (1H, bt); 3.46 (4H, bd); 3.17 (2H, bs); 2.76 (4H, bd, J *10.45 Hz).*
¹³C NMR (DMSO-*d6*, 125 MHz): 151.3; 132.3; 127.8; 123.9; 112.4 (2C); 112.0; 102.7; 80.5; 79.7 (2C); 78.1; 76.0 (2C); 56.6; 53.5; 42.0 (2C); 23.4.

### Analogue A3 (Formic acid salt) (N-(2-(5-hydroxy-1H-indol-3-yl)ethyl)prop-2-yn-1-aminium formate):

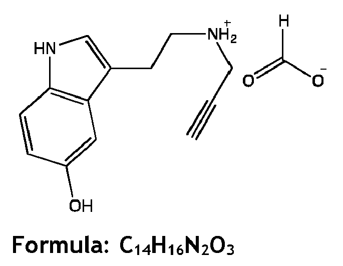

### UPLC: R_{T}: 0.80 - 1.00

¹H NMR (DMSO-*d6*, 500 MHz): 10.51 (1H, s); 8.31 (1H, s (FA)); 7.13 (1H, d, *J 8.55 Hz);* 7.05 (1H, s); 6.82 (1H, s); 6.60 (1H, dd, J *1.75* & *8.5 Hz);* 3.50 (1H, d, *J 1.65 Hz);* 3.19 (1H, s); 2.91 (2H, t, *J 7*.*15 Hz);* 2.78 (2H, t, *J 7.45 Hz).*
¹³C NMR (DMSO-*d6*, 125 MHz): 164.6; 150.6; 131.3; 128.3; 123.6; 112.1; 111.7; 111.2; 102.7; 81.7; 75.3; 48.5; 37.3; 24.9.

### Analogue A4 (3-(2-(di(prop-2-yn-1-yl)amino)ethyl)-1H-indol-5-ol):

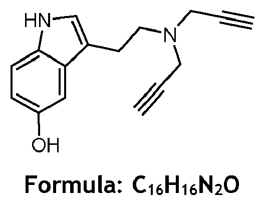

### UPLC: R_{T}: 1.42

¹H NMR (DMSO-*d6*, 500 MHz): 10.46 s); 8.58 bs (OH)); 7.11 d, J 8.5 *Hz);* 7.04 s); 6.82 s); 6.60 dd, J *1.1* & *8.4 Hz);* 3.45 (4H, bs); 3.17 (2H, bs); 2.73 (4H, bs). (+ FA trace)
¹³C NMR (DMSO-*d6*, 125 MHz): 150.6; 131.2; 128.3; 123.4; 112.1; 111.7; 111.6; 102.7; 79.7(2C); 76.1 (2C); 53.5; 42.0 (2C); 23.6. (+ FA trace- 163.9)

### Procedure for the synthesis of A2:

Serotonin hydrochloride (500 mg, 2.35 mmol, 1 eq.) was dissolved in water (9 mL). K₂CO₃ (665 mg, 4.81 mmol, 2.1 eq.) and BOC₂O (538 mg, 2.46 mmol, 1.05 eq.) were added to the solution. The solution was stirred overnight, then extract with DCM. The organic phase was washed with HCl 5% and brine, then dried on MgSO4 and concentrated. The crude was purified by flash chromatography using DCM / MeOH (100/0 to 90/10) as eluent to give the desired product (485 mg), which was engaged in the next step.

Under inert atmosphere, the product of the previous step (485 mg, 1.75 mmol, 1 eq.) was dissolved in dry acetonitrile (5 mL). K₂CO₃ (435 mg, 3.15 mmol, 1.8 eq.) and propargyl bromide (235 µL, 2.1 mmol, 1.2 eq.) were added to the solution. The mixture was stirred and heated at reflux overnight, cooled down to r.t. then filtered with acetonitrile and concentrated. The crude was purified by flash chromatography using cyclohexane / EtOAC (90/10 to 0/100) as eluent.

The product was then dissolved in 1M HCl in EtOAc (10 mL) and stirred for 2 h until the product was predominant in UPLC analysis. The mixture was then concentrated and the crude was directly purified by preparative HPLC to give the desired product after lyophilization.

### Analogue A2 (Formic acid salt) (2-(5-(prop-2-yn-1-yloxy)-1H-indol-3-yl)ethanaminium formate):

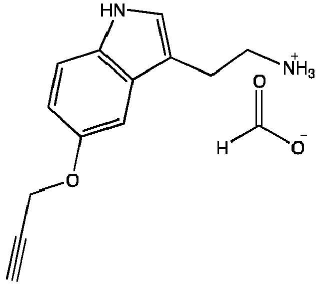

### UPLC: R_{T}: 1.57

¹H NMR (DMSO-*d6*, 500 MHz): 10.82(1H, s); 8.45 (1H, s) 7.26 (1H, d, *J 8 Hz);* 7.18 (1H, d, J *2*.*1 Hz);* 7.13 d, *J 2.1 Hz);* 6.78 dd, *J 8.5* & *2.3 Hz);* 4.77 (2H, d, J *1.8 Hz);* 3.51 (1H, t, *J 2.3 Hz);* 2.94 (4H, m)

### 2. Biological results

*In vivo* studies have been carried out by using 3 mouse models.

### Model 1: Hbb^{Th1/th1}

Hbb^{th1/th1} mice carry a 3.7-kb homozygous spontaneous deletion that eliminates the Hemoglobin Subunit Beta (HBB) gene and 2 kb of the 5' flanking region, including the promoter. On the basis of genetic and hematological criteria, these mice constitute the first animal model of beta-thalassemia. They exhibit iron overload in the spleen, transfusion independent, ineffective erythropoiesis, hepatosplenomegaly, anemia and aberrant erythrocyte morphology (see Dussiot et al., Nature Medicine 2014; 20 (4), 398-407).

### Model 2: Tph1 KO

This model is a peripheral serotonin deficient mice. It is a mouse model of low risk myelodysplastic syndrome, with ineffective erythropoiesis, light anemia, iron overload (spleen), abnormal red blood cells and apoptosis of proerythroblasts (see Côté et al., PNAS, 2003, 100(23), 13525-13530).

### Model 3: Hepcidin KO

Hepcidin, encoded by the *HAMP* gene, is the main regulator of iron homeostasis, and its expression is tightly regulated by signals including iron levels, erythropoietic activity, hypoxia, and inflammation.

It is a mouse model of hemochromatosis. Hepcidin deficient mice progressively develop multivisceral iron overload; plasma iron overcomes transferrin binding capacity, and nontransferrin-bound iron accumulates in various tissues including pancreas and heart (see Nicolas et al., PNAS 2001, 98(15), 8780-8785).

### 2.1 Experimental approach

Mouse models Hbb^{Th1/Th1} with ineffective erythropoiesis and iron overload received intraperitoneal (Ip) injection of A1 or A3 analogues starting on day 0.

Mouse models Tph1 KO with ineffective erythropoiesis and iron overload received intraperitoneal (Ip) injection of A1, A3, A4 analogues or serotonin starting on day 0.

Mouse model of iron overload without ineffective erythropoiesis (Hamp KO) received intraperitoneal (Ip) injection of A3 analogue or serotonin starting on day 0.

Control mice (Wild-type, non-modified, healthy) and a model mice of each type who did not receive any injection were also evaluated.

A complete red blood cells count was achieved on day 1, day 2 and day 5.

On day 5, the animals were sacrificed and histology of their organs and iron measurement in organs was carried out.

### 2.2 Results

### • Model 1(Hbb^{Th1/th1} mice): red blood cells production and iron overload

The evolution over days of the red blood count, the hemoglobin rate and the hematocrit contents in Hbb^{Th1/th1} mice that received either analogue A1 or analogue A3 is illustrated on Figure 1.

It is demonstrated that, analogues A1 and A3 improved hemoglobin (Figure 1B), hematocrit contents (Figure 1C) and RBC count (Figure 1A), thus ameliorating anemia in β-thalassemic mice.

The iron content in different organs (spleen, bone marrow) has been measured on day 5 (after sacrifice) in control mice, and in **Hbb^{Th1/th1}** mices that received A1 or A3. The results are illustrated in Figure 1D.

These results show that analogues A1 or A3 decreased the iron overload in spleen, of **Hbb^{Th1/th1}** mices. The iron is mobilized out of the spleen and relocate into cells of the bone marrow where it is needed for the synthesis of red blood cells.

### • Model 2 (Tph1 KO mice): red blood cells production and iron overload

The evolution over days of the red blood count, the hemoglobin rate, the hematocrit contents, the mean cellular volume (MCV) in Tph1 KO mice that received either analogue A3 or analogue A4 or serotonin is illustrated on Figure 2.

It is demonstrated that, analogues A3 and A4 and serotonin improved hemoglobin (Figure 2B), hematocrit contents (Figure 2C) heme content in red blood cells (Figure 2D), and RBC count (Figure 2A), thus ameliorating anemia in Tph1 KO mice.ln addition, A3 and A4 analogues and serotonin decrease the mean cellular volume (MCV) (Figure 2E). The MCV is a measure of the average volume (size) of red blood cells (RBCs) in a blood sample and an increase in MCV is associated with macrocytic anemia. The results suggest that injection of A3, A4 analogues or serotonin ameliorate the anemic phenotype.

In addition, the iron content in different organs (spleen, bone marrow) has been measured on day 5 (after sacrifice) in control mice, and in Tph1 KO mice that received A3 or A4 analogues or serotonin. The results are illustrated in Figures 2F and 2G. The iron is mobilized out of the spleen and relocate into cells of the bone marrow where it is needed for the synthesis of red blood cells.

### • Model 3 (Hamp KO): iron overload

The iron content was measured in blood and liver in Hamp KO mice and control mice on day 5 (after sacrifice) that received A3 or serotonin. The results are illustrated in Figure 3. The iron level is reduced in the blood and the liver in treated mice. Moreover, the transferrin saturation is also reduced suggesting that iron uptake is reduced.

## Claims

1. Compound of formula (I): 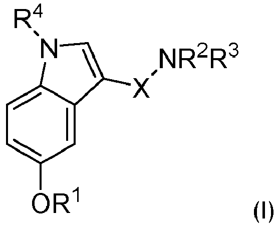 or a pharmaceutically acceptable salt and/or solvate thereof,
wherein
R¹, R², R³ and R⁴ are independently selected in the group consisting of H, optionally substituted C₁-C₂₄alkyl, optionally substituted C₂-C₂₄ alkenyl, optionally substituted C₂-C₂₄ alkynyl, optionally substituted C₃-C₇ cycloalkyl, optionally substituted heterocycloalkyl, optionally substituted aryl and optionally substituted heteroaryl, provided that at least one of R¹, R² and R³ is not H, and
X is selected in the group consisting of C₁-C₁₂ alkyl, O-C₁-C₁₂ alkyl, C(O), C(O)-C₁-C₁₂ alkyl and NH-C(O)-C₁-C₁₂ alkyl.

2. The compound according to claim 1, wherein X is a C₁-C₆ alkyl, preferably X is an ethyl.

3. The compound according to claim 1 or 2, wherein R¹, R² and R³ are independently selected in the group consisting of H, C₁-C₁₂ alkyl, C₂-C₁₂ alkenyl and C₂-C₁₂ alkynyl, said alkyl, alkenyl or alkynyl being optionally substituted with one or more halogens, C₁-C₆ alkyl, aryl, oxo, NH₂, CO₂H or OH.

4. The compound according to any one of claims 1 to 3, wherein R¹, R² and R³ are independently selected in the group consisting of H, C₁-C₆ alkyl, C₂-C₆ alkenyl and C₂-C₆ alkynyl.

5. The compound according to any one of claims 1 to 4, wherein at least one of R¹, R² and R³ is an optionally substituted C₂-C₁₂ alkynyl.

6. The compound according to any one of claims 1 to 5, wherein R⁴ is H.

7. The compound according to any one of claims 1 to 6, being selected from the following compounds:

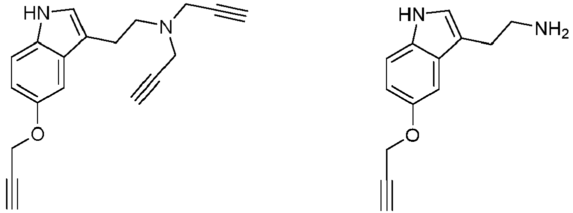

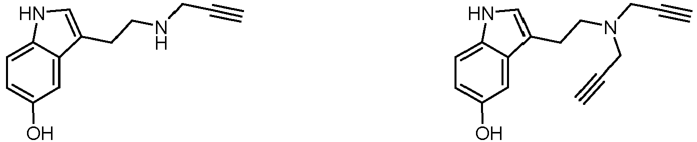

8. A compound according to any one of claims 1 to 7 for use as a drug.

9. The compound for use according to claim 8, for use in preventing or treating iron-associated disorders, in particular iron overload- associated disorders.

10. The compound for use according to claim 9, wherein the iron- associated disorders are iron overload-associated disorders selected among HFE-related hematochromatosis, non HFE-related hematochromatosis, congenital atransferrinenemia, iron-loading associated anemias, chronic liver diseases, chronic inflammation linked to cancer, autoimmune or inflammatory diseases, neurodegeneration with brain iron accumulation- associated diseases and polygenic neurodegenerative- associated diseases.

11. The compound for use according to claim 9 or 10, wherein the iron overload-associated disorders are iron-loading associated anemias, such as thalassemia, myelodysplasia and hematopoietic stem-cell transplantation-associated disorders.

12. A pharmaceutical composition comprising a compound according to any one of claims 1 to 7 and at least one pharmaceutically acceptable excipient.

13. The pharmaceutical composition according to claim 12, for use in preventing or treating iron-associated disorders, in particular iron overload-associated disorders, such as HFE-related hematochromatosis, non HFE-related hematochromatosis, congenital atransferrinenemia, iron-loading associated anemias, chronic liver diseases, chronic inflammation linked to cancer, autoimmune or inflammatory diseases, neurodegeneration with brain iron accumulation- associated diseases and polygenic neurodegenerative-associated diseases.
